# EUROPÄISCHE PATENTSCHRIFT

(11) **EP 1 799 204 B1**
(45) Veröffentlichungstag und Bekanntmachung des Hinweises auf die Patenterteilung: **23.09.2009**
(21) Anmeldenummer: 05801288.1
(22) Anmeldetag: 11.10.2005
(51) Int. Cl.: A61K 31/202, A61K 33/30, A23L 1/30, A23L 1/302, A23L 1/304, A61P 27/02

(54) **OMEGA-3-FETTSÄUREN UND OMEGA-6-FETTSÄUREN ENTHALTENDE ZUSAMMENSETZUNG**
COMPOSITION CONTAINING OMEGA-3 FATTY ACIDS AND OMEGA-6 FATTY ACIDS
COMPOSITIONS CONTENANT DES ACIDES GRAS OMEGA-3 ET OMEGA-6

(30) Priorität: 13.10.2004 DE 202004015931 U
(43) Veröffentlichungstag der Anmeldung: 27.06.2007
(73) Patentinhaber: Dr. GERHARD MANN chem.-pharm. Fabrik GmbH, D-13581 Berlin (DE)
(72) Erfinder: CLAUS-HERZ, Gudrun, 14167 Berlin (DE); BELLMANN, Dr. Günther, 13593 Berlin (DE)
(74) Vertreter: Maiwald, Walter
(86) Internationale Anmeldenummer: PCT/EP2005/055168
(87) Internationale Veröffentlichungsnummer: WO 2006/040324

(56) Entgegenhaltungen:
- EP-A- 0 806 207
- EP-A- 1 275 399
- WO-A-02/39978
- DE-U1-202004 015 931
- FR-A- 2 860 976
- US-B2- 6 506 412
- ANONIMOUS: "Ocuvite omega"[Online] 23. August 2004 (2004-08-23), XP002357668 Gefunden im Internet: URL:https://www.ocuthek.net/imperia/md/con tent/folder/gebrauchsinformationen/ocuvite _omega_pb.pdf> [gefunden am 2005-12-06]
- ANONIMOUS: "Ocuvite Omega"[Online] 17. Dezember 2004 (2004-12-17), XP002357737 Gefunden im Internet: URL:http://www.med1.de/Forum/Augen/120580/ > [gefunden am 2005-12-07]
- "New Products Report"[Online] Januar 2002 (2002-01), XP002357746 Gefunden im Internet: URL:http://www.creativemag.com/npr102.html > [gefunden am 2005-12-06]
- ANONIMOUS: "Ocuvite PreserVision Tablets"[Online] 6. Dezember 2005 (2005-12-06), XP002357747 Gefunden im Internet: URL:http://shop.store.yahoo.com/physlabs/1 21515.html> [gefunden am 2005-12-06]
- PATENT ABSTRACTS OF JAPAN Bd. 2003, Nr. 12, 5. Dezember 2003 (2003-12-05) & JP 2004 357508 A (FANCL CORP), 24. Dezember 2004 (2004-12-24)

## Beschreibung

Die vorliegende Erfindung betrifft eine Omega-6- und Omega-3-Fettsäuren enthaltende Zusammensetzung, die zusätzlich Zinkverbindungen enthält. Die Zusammensetzung kann als Nahrungsergänzungsmittel oder zur ergänzenden bilanzierten Diät zur Behandlung der Symptomatik des trockenen Auges ("dry eye syndrome") verwendet werden.

Jeder fünfte Patient, der eine Augenarztpraxis aufsucht, leidet statistisch gesehen an trockenen Augen. Deutschlandweit wird die Zahl der betroffenen auf ca. 2 Millionen geschätzt. Es ist allgemein bekannt, dass die Augen durch den Sehprozess, beispielsweise durch SAM:AL Bildschirmarbeit, langes Fernsehen, das Tragen von Kontaktlinsen oder durch trockene Heizungs- und Klimaanlagenluft hohen Belastungen ausgesetzt werden. Diese Belastungen äußern sich unter anderem in Brennen, Jucken oder Tränen der Augen. Hierfür ist oft eine Funktionsstörung des Tränenfilms die Ursache, ausgelöst durch zu hohe Verdunstung oder zu geringe Tränenproduktion. Aber auch durch hormonelle Umstellungen im Alter, durch Einnahme von bestimmten Medikamenten (wie zum Beispiel Antibiotika, Antihypertensiva, Antihistaminika, Vasokonstriktoren, Kontrazeptiva, Diuretika oder Antidepressiva) oder durch innere Erkrankungen wie dem Sjögren-Syndrom, Rheuma oder Diabetes können die Benetzungsstörungen des Auges begünstigt werden.

Trockene Augen treten auf, wenn das empfindliche System von Tränenproduktion und Tränenverteilung gestört ist. Häufig ist dieses ein chronisches Phänomen, das einer fortlaufenden Behandlung bedarf. Die Funktionsstörungen des Tränenfilms können sich in einer Vielzahl von Symptomen äußern. Die häufigsten Beschwerden von Patienten, die an trockenem Auge leiden, sind Trockenheitsgefühl, Fremdkörpergefühl, Sandkorngefühl oder Druck auf den Augenlidern.

Eine normale Tränensekretion und ein normaler Tränenfluss sind für die Funktion und das Wohlbefinden des Auges von wesentlicher Bedeutung. Der auf der Hornhaut liegende Tränenfilm erfüllt zahlreiche wichtige Funktionen. Beispielsweise erzeugt er eine glatte Hornhautoberfläche, die sowohl für die optischen Eigenschaften als auf für die Bewegung des Auges und der Lider wichtig ist, er verhindert eine Reizung der Hornhaut durch Austrocknung, er beseitigt Fremdkörper auf mechanischen Wege, zum Beispiel durch Ausschwemmung, und er unterstützt den Stoffwechsel der Hornhaut. Der Tränenfilm besteht aus der inneren Schleimschicht, der mittleren wässrigen Schicht und der äußeren Lipidschicht.

Im Stand der Technik sind Omega-3-Fettsäuren enthaltende Zusammensetzungen bekannt. So offenbart die WO 2004/004599 A3 (ADVANCED VISION RESEARCH) ein Verfahren zur Behandlung eines Zustandes ausgewählt aus der Gruppe umfassend trockene Augen, Entzündung der Meibom-Drüsen, Fehlfunktion der Meibom-Drüsen und trockenem Mund, umfassend die Verabreichung eines Nahrungsergänzungsmittels, welches ein n-6-Fettsäuren enthaltendes Öl und n-3-reiches Öl enthält, worin das n-3-reiche Öl eine hohe Konzentration von Eicosapentaensäure (EPA) und eine hohe Konzentration von Docosahexaensäure (DHA) aufweist.

Die in der WO 2004/004599 A3 offenbarte Lehre zeigt jedoch noch nicht optimale Behandlungsergebnisse bei trockenem Auge.

US 6,506,412 beschreibt die Verwendung von Omega-6 und Omega-3 Fettsäuren, Vitamin A, Magnesium, Vitamin B6, Ascorbinsäure und Mucin zur Behandlung der Trockenen Augen Syndrome.

Es ist daher Aufgabe der vorliegenden Erfindung, eine Zusammensetzung bereitzustellen, die beispielsweise verbesserte Eigenschaften in Bezug auf Entzündungsmediatoren am Auge bietet und/oder deren Inhaltsstoffe kostengünstiger und/oder mit geringerem Aufwand zur Verfügung stehen.

Diese Aufgabe wird durch eine Omega-3-Fettsäuren und Omega-6-Fettsäuren enthaltende Zusammensetzung gelöst, insbesondere geeignet zur Behandlung der Symptomatik des trockenen Auges, wobei die Zusammensetzung zusätzlich Zinkverbindungen, vorzugsweise in Form von Zinksulfat, umfasst.

Es wird angenommen, dass die in der erfindungsgemäßen Zusammensetzung enthaltenen Omega-3- und Omega-6-Fettsäuren im Körper unter anderem zu den Prostaglandinen PGE₁ und PGE₃ metabolisiert werden, wobei wahrscheinlich durch den Zusatz von Zinkverbindungen die erfindungsgemäße Omega-3-Fettsäuren und Omega-6-Fettsäuren enthaltende Zusammensetzung verbesserte Ergebnisse bei der Behandlung der Symptomatik des Trockenen Auges aufweist.

Hierbei hat sich überraschenderweise herausgestellt, dass Omega-3-Fettsäuren mit einer geringeren Konzentration von Eicosapentaensäure (EPA) und/oder einer geringeren Konzentration von Docosahexaensäure (DHA) bevorzugt verwendbar sind, um gute Ergebnisse bei der Behandlung der Symptomatik des Trockenen Auges zu erreichen. Möglicherweise steigert die Zugabe von Zinkverbindungen die Wirkungen der Omega-6-Fettsäuren.

Ohne auf eine bestimmte Theorie festgelegt zu sein, wird vermutet, dass der Zusatz von Zinkverbindungen einen maßgeblichen Einfluss bei der verbesserten Behandlung der Symptomatik des Trockenen Auges bei gleichzeitiger Verwendung von Omega-3-Fettsäuren und Omega-6-Fettsäuren hat.

Ebenfalls scheint insbesondere die Verwendung von Omega-3-Fettsäuren mit einer geringeren Konzentration von Eicosapentaensäure (EPA) und/oder einer geringeren Konzentration von Docosahexaensäure (DHA) in Kombination mit wenigstens einer Zinkverbindung eine überraschend gute synergistische Wirkung bei der Behandlung der Symptomatik des Trockenen Auges zu besitzen.

Weiterhin kann es durch die Verwendung der erfindungsgemäßen Zusammensetzung, umfassend Omega-3- und Omega-6-Fettsäuren in Kombination mit Zinkverbindungen, zu einer gesteigerten Produktion von für die Bildung von Lipiden des Tränenfilms benötigten Vorläuferverbindungen kommen.

Gewichtsangaben im Sinne dieser Erfindung, wenn nicht anders angegeben, beziehen sich auf die jeweilige Zusammensetzung umfassend die Aktivsubstanzen ohne Kapselhülle.

Aktivsubstanzen im Sinne dieser Erfindung sind die Stoffe, Komponenten, Verbindungen und der gleichen, die die Zusammensetzung ohne Kapselhülle ausmachen.

Eine bevorzugte erfindungsgemäße Zusammensetzung weist beispielsweise
≥ 1,68 Gew.-% bis ≤ 83,75 Gew.-%, bevorzugt ≥ 16,75 Gew.-% bis ≤ 67,00 Gew.-%, mehr bevorzugt ≥ 33,50 Gew.-% bis ≤ 50,25 Gew.-%, besonders bevorzugt ≥ 46,90 Gew.-% bis ≤ 48,58 Gew.-% Omega-3-Fettsäuren, als Triglyceride berechnet und bezogen auf das Gesamtgewicht der Zusammensetzung, auf; und/oder
≥ 0,17 Gew.-% bis ≤ 1,68 Gew.-%, bevorzugt ≥ 0,34 Gew.-% bis ≤ 1,34 Gew.-%, mehr bevorzugt ≥ 0,67 Gew.-% bis ≤ 1,01 Gew.-%, besonders bevorzugt ≥ 0,75 Gew.-% bis ≤ 0,92 Gew.-% Omega-6-Fettsäuren, umfassend Gamma-Linolensäure, bezogen auf das Gesamtgewicht der Zusammensetzung, auf.

Besonders bevorzugt ist, wenn die erfindungsgemäße Zusammensetzung beispielsweise ≥ 25 Gew.-% bis ≤ 75 Gew.-%, bevorzugt ≥ 30 Gew.-% bis ≤ 70 Gew.-%, mehr bevorzugt ≥ 40 Gew.-% bis ≤ 60 Gew.-%, besonders bevorzugt ≥ 49 Gew.-% bis ≤ 51 Gew.-% Eicosapentaensäure (EPA), als Triglyceride berechnet und bezogen auf das Gesamtgewicht der Omega-3-Fettsäuren (als Triglyceride berechnet) der Zusammensetzung, aufweist; und/oder
≥ 3 Gew.-% bis ≤ 60 Gew.-%, bevorzugt ≥ 10 Gew.-% bis ≤ 50 Gew.-%, mehr bevorzugt ≥ 20 Gew.-% bis ≤ 40 Gew.-%, besonders bevorzugt ≥ 32 Gew.-% bis ≤ 34 Gew.-% Docosahexaensäure (DHA) (als Triglycerid berechnet) bezogen auf das Gesamtgewicht der Omega-3-Fettsäuren (als Triglyceride berechnet) der Zusammensetzung, aufweist; und/oder ≥ 3 Gew.-% bis ≤ 50 Gew.-%, bevorzugt ≥ 5 Gew.-% bis ≤ 40 Gew.-%, mehr bevorzugt ≥ 10 Gew.-% bis ≤ 30 Gew.-%, besonders bevorzugt ≥ 17 Gew.-% bis ≤ 19 Gew.-% Gamma-Linolensäure (GLA), bezogen auf das Gesamtgewicht der Quelle der Omega-6-Fettsäuren der Zusammensetzung, aufweist.

Geeignete erfindungsgemäß verwendbare Omega-3-Fettsäuren können ausgewählt sein aus der Gruppe umfassend Alpha-Linolensäure, Stearidonsäure, Eicosapentaensäure, Docosapentaensäure und/oder Docosahexaensäure.

Geeignete erfindungsgemäß verwendbare Omega-6-Fettsäuren können ausgewählt sein aus der Gruppe umfassend Linolsäure, Gamma-Linolensäure und/oder Dihomo-Gamma-Linolensäure.

Die erfindungsgemäße Zusammensetzung weist als Zinkverbindungen vorzugsweise Zink in Form von Zinksulfat auf.

Als Zinkverbindungen lassen sich grundsätzlich alle Zinkverbindungen einsetzen, solange sie verträglich sind. Besonders geeignet sind gesundheitsverträgliche anorganische und/oder organische Salze des Zinks. Beispielsweise lassen sich Zinksulfat-Monohydrat, Zinksulfat-Septahydrat, Zinkhistidinat-Dihydrat, Zinkchlorid, Zinkaspartat, Zinkgluconat, Zinkorotat und/oder Zinkoxid erfindungsgemäß verwenden.

Bevorzugt werden der Zusammensetzung Zinkverbindungen in solchen Mengen zugesetzt, dass sie ≥ 0,01 mg bis ≤ 10,00 mg, bevorzugt ≥ 1,00 mg bis ≤ 6,00 mg, mehr bevorzugt ≥ 2,00 mg bis ≤ 4,00 mg, besonders bevorzugt ≥ 3,30 mg bis ≤ 3,40 mg Zink enthält. Diese Angaben sind vorzugsweise bezogen auf eine Einzeldosis der Zusammensetzung. In weiteren bevorzugten Ausführungsformen enthält die Zusammensetzung Zink im Bereich von > 2,5 mg bis < 5 mg, bezogen auf eine Einzeldosis der Zusammensetzung.

Beispielsweise können der erfindungsgemäßen Zusammensetzung Zinkverbindungen in solchen Mengen zugesetzt werden, dass sie ≥ 0,01 Gew.-% bis ≤ 1,68 Gew.-%, bevorzugt ≥ 0,17 Gew.-% bis ≤ 1,01 Gew.-%, mehr bevorzugt ≥ 0,34 Gew.-% bis ≤ 0,67 Gew.-%, besonders bevorzugt ≥ 0,55 Gew.-% bis ≤ 0,57 Gew.-% Zink, bezogen auf das Gesamtgewicht der Zusammensetzung, enthält.

Vorzugsweise enthält die Zusammensetzung wenigstens ein Vitamin. In einer bevorzugten Ausführungsform der vorliegenden Erfindung enthält die Zusammensetzung wenigstens ein Vitamin, ausgewählt aus der Gruppe umfassend Vitamin E, Vitamin C, Vitamin B, wobei B6 und/oder Vitamin B12 bevorzugt sind. Das fettlösliche Vitamin E kann unter anderem als Antioxidans wirken und so die in der Zusammensetzung enthaltenen Omega-3- und Omega-6-Fettsäuren gegen Oxidation schützen. Sowohl der Zusatz von Vitamin C als auch insbesondere von Vitamin B6 kann eine weitere Verbesserung der Wirkung der erfindungsgemäßen Zusammensetzung bewirken.

Weitere Vorteile der erfindungsgemäßen Zusammensetzung können insbesondere durch die Verwendung von Vitamin B6 und/oder Vitamin B 12 in Kombination mit Omega-3- und Omega-6-Fettsäuren und Zink verwirklicht werden. Diese Kombination kann vorteilhafter Weise eine die Tränenproduktion positiv beeinflussende Wirkung aufweisen. Es wird angenommen, dass die Verwendung von Vitamin B6 und/oder Vitamin B12 in Kombination mit Omega-3- und Omega-6-Fettsäuren und Zink dazu beitragen kann, den natürlichen Tränenfilm im Auge zu erhalten und eine Versorgung des Auges mit natürlicher Feuchtigkeit zu verbessern.

Für eine weitere Verbesserung der Wirkung der Zusammensetzung hat sich für eine Einzeldosis der Zusammensetzung ein Gehalt von ≥ 0,1 mg bis ≤ 1,0 mg Vitamin B6 als günstig erwiesen. Als weiterhin geeigneter Gehalt haben sich von ≥ 0,3 mg bis ≤ 0,8 mg Vitamin B6 bezogen auf eine Einzeldosis herausgestellt. Bevorzugt wird ein Gehalt von ≥ 0,5 mg bis ≤ 0,75 mg, besonders bevorzugt ≥ 0,6 mg bis ≤ 0,7 mg Vitamin B6 bezogen auf eine Einzeldosis der Zusammensetzung.

Es können weiterhin Ausführungsformen bevorzugt sein, bei welchen die Zusammensetzung weiterhin Vitamin B12 enthält. Es konnte im Rahmen der Untersuchungen festgestellt werden, dass ein Gehalt an Vitamin B 12, der oberhalb von 10 µg für die Einzeldosis liegt, eine ungünstige Veränderung der Verträglichkeit der Zusammensetzung bewirkt. Vorzugsweise enthält die Zusammensetzung bezogen auf eine Einzeldosis ≥ 0,01 µg bis ≤ 1,0 µg, bevorzugt ≥ 0,15 µg bis ≤ 0,7 µg, mehr bevorzugt ≥ 0,25 µg bis ≤ 0,5 µg, besonders bevorzugt ≥ 0,32 µg bis ≤ 0,34 µg Vitamin B 12.

In bevorzugten Ausführungsformen enthält die Zusammensetzung weiterhin ≥ 0,1 mg bis ≤ 1,0 mg, bevorzugt ≥ 0,3 mg bis ≤ 0,8 mg, mehr bevorzugt ≥ 0,5 mg bis ≤ 0,75 mg, besonders bevorzugt ≥ 0,6 mg bis ≤ 0,7 mg Vitamin B6; und/oder
≥ 0,01 µg bis ≤ 1,0 µg, bevorzugt ≥ 0,15 µg bis ≤ 0,7 µg, mehr bevorzugt ≥ 0,25 µg bis ≤ 0,5 µg, besonders bevorzugt ≥ 0,32 µg bis ≤ 0,34 µg Vitamin B12.

Weiterhin hat sich herausgestellt, dass durch eine zusätzliche Verwendung von Vitamin C gegebenenfalls eine weitere positive Verbesserung der Zusammensetzung erzielt werden kann. In einer weiteren bevorzugten Ausführungsform der vorliegenden Erfindung enthält die Zusammensetzung weiterhin ≥ 1 mg bis ≤ 50 mg, bevorzugt ≥ 10 mg bis ≤ 40 mg, mehr bevorzugt ≥ 15 mg bis ≤ 30 mg, besonders bevorzugt ≥ 19 mg bis ≤ 21 mg Vitamin C.

In besonders bevorzugten Ausführungsformen der Zusammensetzung umfasst die Zusammensetzung Omega-3-Fettsäuren, Gamma-Linolensäure, Vitamin C, Vitamin E, Vitamin B6, Vitamin B 12 und/oder Zink.

In weiterhin besonders bevorzugten Ausführungsformen der Zusammensetzung umfasst die Zusammensetzung Fischöl, Borretschsamenöl, Vitamin C, Vitamin E, Vitamin B6, Vitamin B 12 und/oder Zink.

In einer bevorzugten Ausführungsform der vorliegenden Erfindung umfasst die Zusammensetzung für eine Einzeldosis:
- ≥ 10 mg bis ≤ 500 mg, bevorzugt ≥ 100 mg bis ≤ 400 mg, mehr bevorzugt ≥ 200 mg bis ≤ 3 00 mg, besonders bevorzugt ≥ 280 mg bis ≤ 290 mg Omega-3-Fettsäuren als Triglyceride berechnet; und/oder
- ≥ 1 mg bis ≤ 10 mg, bevorzugt ≥ 2 mg bis ≤ 8 mg, mehr bevorzugt ≥ 4 mg bis ≤ 6 mg, besonders bevorzugt ≥ 4,5 mg bis ≤ 5,5 mg Gamma-Linolensäure; und/oder
- ≥ 1 mg bis ≤ 5 mg, bevorzugt ≥ 2 mg bis ≤ 4,5 mg, mehr bevorzugt ≥ 3 mg bis ≤ 4 mg, besonders bevorzugt ≥ 3,1 mg bis ≤ 3,5 mg Vitamin E; und/oder
- ≥ 0,01 mg bis ≤ 5 mg, bevorzugt ≥ 0,1 mg bis ≤ 3 mg, mehr bevorzugt ≥ 0,5 mg bis ≤ 2 mg, besonders bevorzugt ≥ 0,9 mg bis ≤ 1,1 mg gemischtes Tocopherol-Konzentrat; und/oder
- ≥ 1 mg bis ≤ 50 mg, bevorzugt ≥ 10 mg bis ≤ 40 mg, mehr bevorzugt ≥ 15 mg bis ≤ 30 mg, besonders bevorzugt ≥ 19 mg bis ≤ 21 mg Vitamin C; und/oder
- ≥ 0,1 mg bis ≤ 1,0 mg, bevorzugt ≥ 0,3 mg bis ≤ 0,8 mg, mehr bevorzugt ≥ 0,5 mg bis ≤ 0,75 mg, besonders bevorzugt ≥ 0,6 mg bis ≤ 0,7 mg Vitamin B6; und/oder
- ≥ 0,01 µg bis ≤ 1,0 µg, bevorzugt ≥ 0,15 µg bis ≤ 0,7 µg, mehr bevorzugt ≥ 0,25 µg bis ≤ 0,5 µg, besonders bevorzugt ≥ 0,32 µg bis ≤ 0,34 µg Vitamin B 12; und/oder
- ≥ 0,1 mg bis ≤ 10 mg, bevorzugt ≥ 1,5 mg bis ≤ 5 mg, mehr bevorzugt ≥ 2 mg bis ≤ 4 mg, besonders bevorzugt ≥ 3,3 mg bis ≤ 3,4 mg Zink.

In einer besonders bevorzugten Ausführungsform der Zusammensetzung umfasst die Zusammensetzung bezogen auf eine Einzeldosis 285 mg Omega-3-Fettsäuren als Triglyceride berechnet, 5 mg Gamma-Linolensäure, 20 mg Vitamin C, 3,5 mg Vitamin E, 0,67 mg Vitamin B6, 0,34 µg Vitamin B12 und/oder 3,33 mg Zink.

In einer weiterhin besonders bevorzugten Ausführungsform der Zusammensetzung umfasst die Zusammensetzung bezogen auf eine Einzeldosis 475 mg Fischöl, 29,4 mg Borretschsamenöl, 20 mg Vitamin C, 3,5 mg Vitamin E, 0,67 mg Vitamin B6, 0,34 µg Vitamin B12 und/oder 3,33 mg Zink.

In einer ganz besonders bevorzugten Ausführungsform der Zusammensetzung umfasst die Zusammensetzung bezogen auf eine Tagesdosis 855 mg Omega-3-Fettsäuren als Triglyceride berechnet, 15 mg Gamma-Linolensäure, 60 mg Vitamin C, 10 mg Vitamin E, 2 mg Vitamin B6, 1 µg Vitamin B12 und/oder 10 mg Zink.

In einer weiterhin ganz besonders bevorzugten Ausführungsform der Zusammensetzung umfasst die Zusammensetzung bezogen auf eine Tagesdosis 1425 mg Fischöl, 88 mg Borretschsamenöl, 60 mg Vitamin C, 10 mg Vitamin E, 2 mg Vitamin B6, 1 µg Vitamin B12 und/oder 10 mg Zink.

Die erfindungsgemäße Zusammensetzung scheint einen Engpass in der Biosynthese von PGE₁ und PGE₃ zu vermeiden, wobei insbesondere der Zusatz der Omega-3- und Omega-6-Fettsäuren in Kombination von Zinksalz(en) sowie von Vitamin C und B-Vitaminen eine deutliche Verbesserung bei der Behandlung bzw. diätetischen Behandlung der Symptomatik des Trockenen Auges aufweisen.

Im Sinne dieser Erfindung kann das Merkmal Fischöl Öl aus Fischen und/oder Meerestieren umfassen.

Die erfindungsgemäße Zusammensetzung kann vorzugsweise die Omega-3-Fettsäuren in Form von Fischöl-Triglyceriden vorliegen haben. Die Omega-3-Fettsäure liegt am meisten bevorzugt als Eicosapentaensäure und/oder Docosahexaensäure vor, die vorzugsweise in folgenden Ölen wie Rapsöl, Leinsamen oder deren Öl und/oder Fischöl erhältlich sind. Die Omega-6-Fettsäure kann Gamma-Linolensäure sein, welche vorzugsweise aus Borretschsamenöl, Nachtkerzenöl und/oder Kernöl von schwarzen Johannisbeeren erhältlich ist. Weiterhin kann die erfindungsgemäße Zusammensetzung Hilfsstoffe aufweisen, die aus der Gruppe umfassend Glycerinmonostearat, Lecithin und/oder Gelatine ausgewählt sind.

In einer bevorzugten erfindungsgemäßen Ausführungsforin beträgt das Gewichtsverhältnis der Omega-3-Fettsäuren als Triglyceride berechnet und der Gamma-Linolensäure ≥ 3:1 bis ≤ 200:1, bevorzugt ≥ 20:1 bis ≤ 100:1, mehr bevorzugt ≥ 50:1 bis ≤ 60:1, besonders bevorzugt ≥ 56:1 bis ≤ 58:1. Es wird vermutet, dass die Omega-3-Fettsäure Eicosapentaensäure mit der aus der Omega-6-Fettsäurenreihe stammenden Dihomo-Gamma-Linolensäure um das Enzym Delta-5-Desaturase konkurriert. Somit sorgt der im Vergleich zum Stand der Technik große Überschuss an Eicosapentaensäure vermutlich nicht nur für eine Synthese von PGE₃, sondern auch möglicherweise indirekt über die Konkurrenz um die Delta-5-Desaturase für eine Verringerung der Synthese der unerwünschten Arachidonsäure und infolgedessen wahrscheinlich für eine Verringerung der Synthese des unerwünschten PGE₂. Weiterhin scheint dieses dazu zu führen, dass mehr Dihomo-Gamma-Linolensäure über die Cyclooxygenase in das erwünschte PGE₃ überführt wird. Wobei hier der Zusatz von Zinksalzen und gegebenenfalls von Vitaminen einen noch ungeklärten positiven synergistischen Einfluss hat.

Somit führt die erfindungsgemäße Zusammensetzung in der Gesamtheit ihrer Komponenten zu einer positiven Beeinflussung der relativen Verhältnisse der Entzündungsmediatoren, das heißt der Verhältnisse der erwünschten entzündungshemmenden und die Tränensekretion fördernden PGE₁ und PGE₃ einerseits und dem unerwünschten entzündungsfördernden PGE₂ andererseits.

Vorteilhaft ist darüber hinaus, dass die Dosierungen der erfindungsgemäßen Zusammensetzung keine schädigenden Nebenwirkungen verursachen und zur diätetischen Vorbeugung und/oder zur Behandlung von Augenkrankheiten, bevorzugt zur Behandlung der Symptomatik des trockenen Auges, dienen können. Ein positiver Effekt zeigt sich insbesondere durch eine ergänzende bilanzierte Diät oder durch eine Verabreichung als Nahrungsergänzungsmittel bei Mangelzuständen und der Vorbeugung einer Entstehung einer Mangelsituation, die zu einer Symptomatik des trockenen Auges führen kann.

Die Omega-3-Fettsäuren enthaltende Zusammensetzung kann fest-, flüssig und/oder gelförmig vorliegen; vorzugsweise liegt die Zusammensetzungen in Darreichungsformen vor, die aus der Gruppe umfassend Tabletten, Filmtabletten, Dragees, Kapseln, Pulver, Granulat, Lösungen und/oder Brausetabletten ausgewählt ist.

Die Stoffe werden bevorzugt in einer für die gewählte Darreichungsform verarbeitbaren Form eingesetzt. Bevorzugt weist die Omega-3-Fettsäuren enthaltende Zusammensetzung Vitamin E als (R, R, R)-Alpha-Tocopherol und/oder als gemischtes Tocopherol-Konzentrat auf. Bevorzugt weist die Zusammensetzung Vitamin C als Calciumascorbat auf. In besonders bevorzugten Ausführungsformen weist die Omega-3-Fettsäuren enthaltende Zusammensetzung Fischöl-Triglyceride, Borretschsamenöl, (R, R, R)-Alpha-Tocopherol, gemischtes Tocopherol-Konzentrat, Calciumascorbat, Pyridoxinhydrochlorid, Zinksulfat-Monohydrat und Cyanocobalamin, Glycerolmonostearat, Lecithin und/oder Gelatine auf.

Bevorzugt liegt die Omega-3-Fettsäuren enthaltende Zusammensetzung, insbesondere als Nahrungsergänzungsmittel, in Form einer Kapsel, insbesondere als Gelatinekapsel vor. Vorzugsweise umfasst die Kapsel eine Kapselhülle, welche ein Gewicht von ≥ 50 mg bis ≤ 500 mg, bevorzugt ≥ 100 mg bis ≤ 400 mg, mehr bevorzugt ≥ 218 mg bis ≤ 256 mg, besonders bevorzugt ≥ 237 mg bis ≤ 238 mg aufweist. Die Kapselhülle kann Hilfsstoffe aufweisen, die aus der Gruppe umfassend Glycerin, Gelatine und/oder Farbstoffe, z. B. rotes Eisenoxid, ausgewählt sind. Weiterhin geeignete Farbstoffe sind bevorzugt ausgewählt aus der Gruppe umfassend Triarylmethan-Farbstoffe, vorzugsweise Brilliantblau, Azofarbstoffe, vorzugsweise Allurarot, und/oder Titandioxid.

Weiterhin weist in einer bevorzugten Ausführungsform der erfindungsgemäßen Zusammensetzung der Inhalt der Darreichungsform ein Gesamtgewicht von ≥ 1 mg bis ≤ 1000 mg, bevorzugt ≥ 200 mg bis ≤ 800 mg, mehr bevorzugt ≥ 400 mg bis ≤ 700 mg, besonders bevorzugt ≥ 596 mg bis ≤ 598 mg auf. In einer weiterhin bevorzugten Ausführungsform der erfindungsgemäßen Zusammensetzung weist der Inhalt der Darreichungsform, beispielsweise der Inhalt einer Kapsel, ein Gesamtgewicht von ≥ 600 mg bis ≤ 605 mg, besonders bevorzugt von 602 mg, auf.

Die Tagesdosis der erfindungsgemäßen Zusammensetzung kann eine oder mehrere Einzeldosen betragen. Beispielsweise kann die Tagesdosis 3 Einzeldosen betragen.

Der Gegenstand der vorliegenden Erfindung wird anhand der nachstehenden Beispiele 1 bis 12 weiter erläutert.

### BEISPIEL 1

Eine Einzeldosis enthält:
- Fischöl 475 mg, davon
- Omega-3-Fettsäuren (berechnet als Triglyceride) 285 mg
- Borretschsamenöl 29,4 mg, davon
- Gamma-Linolensäure 5 mg
- (R, R, R)-Alpha-Tocopherol-Konzentrat 3,334 mg
- Ascorbinsäure (als Calciumascorbat) 20 mg
- Vitamin B6 (als Pyridoxinhydrochlorid) 0,667 mg
- Zink (als Zinksulfat-Monohydrat) 3,34 mg
- Cyanocobalamin 0,33 µg
- Glycerinmonostearat
- Lecithin
- Gelatine ad 597 mg

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 2

Eine Kapselhülle enthält:
- Glycerin
- Gelatine
- Eisenoxid, rot ad 237,2 mg

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 3

Eine empfohlene Tagesdosis von 3 Kapseln enthält:
- Fischöl 1425 mg, davon
- Omega-3-Fettsäuren (berechnet als Triglyceride) 855 mg
- Borretschsamenöl 88 mg, davon
- Gamma-Linolensäure 15 mg
- (R, R, R)-Alpha-Tocopherol-Konzentrat 10 mg
- Ascorbinsäure (als Calciumascorbat) 60 mg
- Vitamin B6 (als Pyridoxinhydrochlorid) 2 mg
- Zink (als Zinksulfat-Monohydrat) 10 mg
- Cyanocobalamin 1 µg
- Glycerinmonostearat
- Lecithin
- Gelatine ad 1791 mg

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 4

Die 3 Kapselhüllen der empfohlenen Tagesdosis enthalten:
- Glycerin
- Gelatine
- Eisenoxid, rot ad 712 mg

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 5

Eine Einzeldosis enthält:
- Fischöl 475 mg, davon
- Omega-3-Fettsäuren (berechnet als Triglyceride) 285 mg
- Borretschsamenöl 29,4 mg, davon
- Gamma-Linolensäure 5 mg
- (R, R, R)-Alpha-Tocopherol-Konzentrat 3,334 mg
- Ascorbinsäure (als Calciumascorbat) 20 mg
- Vitamin B6 (als Pyridoxinhydrochlorid) 0,667 mg
- Zink (als Zinksulfat-Monohydrat) 3,34 mg
- Cyanocobalamin 0,33 µg wobei Glycerinmonostearat, Lecithin, Gelatine ad 602 mg zugefügt sind

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 6

Eine Kapselhülle von 237,2 mg gebildet aus:
- Glycerin
- Gelatine
- Eisenoxid, rot

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 7

Eine Einzeldosis enthält:
- Fischöl 475 mg, davon
- Omega-3-Fettsäuren (berechnet als Triglyceride) 285 mg
- Borretschsamenöl 29,4 mg, davon
- Gamma-Linolensäure 5 mg
- (R, R, R)-Alpha-Tocopherol-Konzentrat 3,334 mg
- Ascorbinsäure (als Calciumascorbat) 20 mg
- Vitamin B6 (als Pyridoxinhydrochlorid) 0,667 mg
- Zink (als Zinksulfat-Monohydrat) 3,34 mg
- Cyanocobalamin 0,33 µg wobei Glycerinmonostearat, Lecithin, Gelatine ad 602 mg zugefügt sind

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 8

Eine Kapselhülle von 237,87 mg gebildet aus:
- Glycerin
- Gelatine
- Brilliantblau

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 9

Eine empfohlene Tagesdosis von 3 Kapseln enthält:
- Fischöl 1425 mg, davon
- Omega-3-Fettsäuren (berechnet als Triglyceride) 855 mg
- Borretschsamenöl 88 mg, davon
- Gamma-Linolensäure 15 mg
- (R, R, R)-Alpha-Tocopherol-Konzentrat 10 mg
- Ascorbinsäure (als Calciumascorbat) 60 mg
- Vitamin B6 (als Pyridoxinhydrochlorid) 2 mg
- Zink (als Zinksulfat-Monohydrat) 10 mg
- Cyanocobalamin 1 µg wobei Glycerinmonostearat, Lecithin, Gelatine ad 1806 mg zugefügt sind

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 10

Die 3 Kapselhüllen der empfohlenen Tagesdosis von insgesamt 712 mg sind gebildet aus:
- Glycerin
- Gelatine
- Eisenoxid, rot

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 11

Eine empfohlene Tagesdosis von 3 Kapseln enthält:
- Fischöl 1425 mg, davon
- Omega-3-Fettsäuren (berechnet als Triglyceride) 855 mg
- Borretschsamenöl 88 mg, davon
- Gamma-Linolensäure 15 mg
- (R, R, R)-Alpha-Tocopherol-Konzentrat 10 mg
- Ascorbinsäure (als Calciumascorbat) 60 mg
- Vitamin B6 (als Pyridoxinhydrochlorid) 2 mg
- Zink (als Zinksulfat-Monohydrat) 10 mg
- Cyanocobalamin 1 µg wobei Glycerinmonostearat, Lecithin, Gelatine ad 1806 mg zugefügt sind

Zusätzlich zu den angegebenen Mengen sind weitere übliche Hilfsstoffe wie Stabilisatoren und/oder Stabilitätszuschläge und/oder Produktionszuschläge enthalten.

### BEISPIEL 12

Die 3 Kapselhüllen der empfohlenen Tagesdosis von insgesamt 713,6 mg sind gebildet aus:
- Glycerin
- Gelatine
- Brilliantblau

Zusätzlich zu den angegebenen Mengen sind weitere übliche Hilfsstoffe wie Stabilisatoren und/oder Stabilitätszuschläge und/oder Produktionszuschläge enthalten.

Soweit nicht anders angegeben, beziehen sich die Gewichtsangaben auf die jeweils angegebene Verbindung als solche.

## Patentansprüche

1. Omega-3-Fettsäuren und Omega-6-Fettsäuren enthaltende Zusammensetzung zur Behandlung der Symptomatik des Trockenen Auges, **dadurch gekennzeichnet, dass** die Zusammensetzung Zinkverbindungen, vorzugsweise in Form von Zinksulfat, umfasst.

2. Zusammensetzung nach Anspruch 1, worin die Zusammensetzung wenigstens ein Vitamin, ausgewählt aus der Gruppe umfassend Vitamin E, Vitamin C, Vitamin B, vorzugsweise Vitamin B6 und/oder Vitamin B12, aufweist.

3. Zusammensetzung nach Ansprüchen 1 bis 2, worin die Omega-3-Fettsäuren als Fischöl-Triglyceride vorliegen.

4. Zusammensetzung nach Ansprüchen 1 bis 3, worin die Omega-3-Fettsäure Eicosapentaensäure ist, vorzugsweise erhältlich aus Speiseöl, Rapsöl, Leinsamen und/oder Fischöl.

5. Zusammensetzung nach Ansprüchen 1 bis 4, worin die Omega-3-Fettsäure Docosahexaensäure ist.

6. Zusammensetzung nach Ansprüchen 1 bis 5, worin die Omega-6-Fettsäure Gamma-Linolensäure ist, vorzugsweise erhältlich aus Borretschsamenöl, Nachtkerzenöl und/oder Kemöl von schwarzen Johannisbeeren.

7. Zusammensetzung nach Ansprüchen 1 bis 6, worin die Zusammensetzung, bezogen auf eine Einzeldosis der Zusammensetzung, umfasst:
• ≥ 10 mg bis ≤ 500 mg, bevorzugt ≥ 100 mg bis ≤ 400 mg, mehr bevorzugt ≥ 200 mg bis ≤ 300 mg, besonders bevorzugt ≥ 280 mg bis ≤ 290 mg Omega-3-Fettsäuren als Triglyceride berechnet; und/oder
• ≥ 1 mg bis ≤ 10 mg, bevorzugt ≥ 2 mg bis ≤ 8 mg, mehr bevorzugt ≥ 4 mg bis ≤ 6 mg, besonders bevorzugt ≥ 4,5 mg bis ≤ 5,5 mg Gamma-Linolensäure; und/oder
• ≥ 1 mg bis ≤ 5 mg, bevorzugt ≥ 2 mg bis ≤ 4,5 mg, mehr bevorzugt ≥ 3 mg bis ≤ 4 mg, besonders bevorzugt ≥ 3,1 mg bis ≤ 3,5 mg Vitamin E; und/oder
• ≥ 0,01 mg bis ≤ 5 mg, bevorzugt ≥ 0,1 mg bis ≤ 3 mg, mehr bevorzugt ≥ 0,5 mg bis ≤ 2 mg, besonders bevorzugt ≥0,9 mg bis ≤ 1,1 mg gemischtes Tocopherol-Konzentrat; und/oder
• ≥ 1 mg bis ≤ 50 mg, bevorzugt ≥ 10 mg bis ≤ 40 mg, mehr bevorzugt ≥ 15 mg bis ≤ 30 mg, besonders bevorzugt ≥ 19 mg bis ≤ 21 mg Vitamin C; und/oder
• ≥ 0,1 mg bis ≤ 1,0 mg, bevorzugt ≥ 0,3 mg bis ≤ 0,8 mg, mehr bevorzugt ≥ 0,5 mg bis ≤ 0,75 mg, besonders bevorzugt ≥ 0,6 mg bis ≤ 0,7 mg Vitamin B6; und/oder
• ≥ 0,01 µg bis ≤ 1,0 µg, bevorzugt ≥ 0,15 µg bis ≤ 0,7 µg, mehr bevorzugt ≥ 0,25 µg bis ≤ 0,5 µg, besonders bevorzugt ≥ 0,32 µg bis ≤ 0,34 µg Vitamin B 12; und/oder
• ≥ 0,1 mg bis ≤ 10 mg, bevorzugt ≥ 1,5 mg bis ≤ 5 mg, mehr bevorzugt ≥ 2 mg bis ≤ 4 mg, besonders bevorzugt ≥ 3,3 mg bis ≤ 3,4 mg Zink.

8. Zusammensetzung nach Ansprüchen 1 bis 7, worin das Gewichtsverhältnis der Omega-3-Fettsäuren als Triglyceride berechnet und der Gamma-Linolensäure ≥ 3:1 bis ≤ 200:1, bevorzugt ≥ 20:1 bis ≤ 100:1, mehr bevorzugt ≥ 50:1 bis ≤ 60:1, besonders bevorzugt ≥56:1 bis ≤ 58:1 beträgt.

9. Zusammensetzung nach Ansprüchen 1 bis 8, worin die Zusammensetzung fest-, flüssig und/oder gelförmig vorliegt; vorzugsweise liegt die Zusammensetzung in Darreichungsformen ausgewählt aus der Gruppe umfassend Tabletten, Filmtabletten, Dragees, Kapseln, Pulver, Granulat, Lösungen und/oder Brausetabletten vor.

10. Zusammensetzung nach Ansprüchen 1 bis 9, worin der Inhalt der Darreichungsform ein Gesamtgewicht von ≥ 1 mg bis ≤ 1000 mg, bevorzugt ≥ 200 mg bis ≤ 800 mg, mehr bevorzugt ≥ 400 mg bis ≤ 700 mg, besonders bevorzugt ≥ 596 mg bis ≤ 598 mg aufweist.

11. Zusammensetzung nach Ansprüchen 1 bis 10, worin die Zusammensetzung Hilfsstoffe ausgewählt aus der Gruppe umfassend Glycerinmonostearat, Lecithin und/oder Gelatine aufweist.

12. Zusammensetzung nach Ansprüchen 1 bis 11, worin deren Darreichungsform in Kapselform ist und/oder die Kapselhülle ein Gewicht von ≥ 50 mg bis ≤ 500 mg, bevorzugt ≥ 100 mg bis ≤ 400 mg, mehr bevorzugt ≥ 218 mg bis ≤ 256 mg, besonders bevorzugt ≥ 237 mg bis ≤ 238 mg aufweist.

13. Zusammensetzung nach Ansprüchen 1 bis 12, worin die Kapselhülle Hilfsstoffe ausgewählt aus der Gruppe umfassend Glycerin, Gelatine und/oder Farbstoffe, z. B. rotes Eisenoxid, aufweist.

14. Verwendung der Zusammensetzung nach Ansprüchen 1 bis 13 zur Herstellung eines Mittels zur diätetischen Vorbeugung und/oder Behandlung von Augenkrankheiten, bevorzugt zur Behandlung der Symptomatik des Trockenen Auges.

15. Verwendung der Zusammensetzung nach Ansprüchen 1 bis 14 als Nahrungsergänzungsmittel und/oder Mittel zur ergänzenden bilanzierten Diät.

16. Mittel, Nahrungsergänzungsmittel oder Mittel zur ergänzenden bilanzierten Diät, insbesondere Lebensmittel, umfassend eine Zusammensetzung gemäß der Ansprüche 1 bis 15.

## Claims

1. Composition containing omega-3 fatty acids and omega-6 fatty acids for the treatment of dry eye syndrome, **characterized in that** the composition comprises zinc compounds, preferably in form of zinc sulphate.

2. Composition of claim 1, wherein the composition has at least one vitamin selected from the group comprising vitamin E, vitamin C, vitamin B, preferably vitamin B6 and/or vitamin B 12.

3. Composition of claims 1 to 2, wherein the omega-3-fatty acids are present as fish oil triglycerides.

4. Composition of claims 1 to 3, wherein the omega-3 fatty acid is eicosapentaenoic acid, preferably obtainable from edible oil, rape oil, linseed and/or fish oil.

5. Compositions of claims 1 to 4, wherein the omega-3 fatty acid is docosahexaenoic acid.

6. Composition of claims 1 to 5, wherein the omega-6 fatty acid is gamma-linolenic acid, preferably obtainable from borage seed oil, evening primrose oil and/or core oil from black currant.

7. Composition of claims 1 to 6, wherein the composition, based on a single dose of the composition, comprises:
• **≥** 10 mg to ≤500 mg, preferably ≥ 100 mg to ≤ 400 mg, more preferably ≥ 200 mg to ≤ 300 mg, especially preferably ≥ 280 mg to ≤ 290 mg omega-3 fatty acid calculated as triglycerides; and/or
• ≥ 1 mg to ≤10 mg, preferably ≥ 2 mg to < 8 mg, more preferably ≥ 4 mg to ≤ 6 mg, especially preferably ≥ 4,5 mg to ≤ 5,5 mg gamma-linolenic acid; and/or
• ≥ 1 mg to ≤ 5 mg, preferably ≥ 2 mg to ≤ 4,5 mg, more preferably ≥ 3 mg to ≤ 4 mg, especially preferably ≥ 3,1 mg to < 3,5 mg vitamin E; and/or
• ≥ 0,01 mg to ≤ 5 mg, preferably ≥ 0,1 mg to < 3 mg, more preferably ≥ 0,5 mg to ≤ 2 mg, especially preferably ≥ 0,9 mg to ≤ 1,1 mg mixed concentrate of tocopherol; and/or
• ≥ 1 mg to ≤ 50 mg, preferably ≥ 10 mg to < 40 mg, more preferably ≥ 15 mg to ≤ 30 mg, especially preferably ≥ 19 mg to < 21 mg vitamin C; and/or
• ≥ 0,1 mg to < 10 mg, preferably ≥ 0,3 mg to < 0,8 mg, more preferably ≥ 0,5 mg to ≤ 0,75 mg, especially preferably ≥ 0,6 mg to ≤ 0,7 mg vitamin B6; and/or
• ≥ 0,01 µg to ≤ 1,0 µg, preferably ≥ 0,15 µg to ≤ 0,7 µg, more preferably ≥ 0,25 µg to < 0,5 µg, especially preferably ≥ 0,32 µg to ≤ 0,34 µg vitamin B12; and/or
• ≥ 0,1 mg to ≤ 10 mg, preferably ≥ 1,5 mg to ≤ 5 mg, more preferably ≥ 2 mg to ≤ 4 mg, especially preferably ≥ 3,3 mg to < 3,4 mg zinc.

8. Composition of claims 1 to 7, wherein the weight ratio of the omega-3 fatty acids calculated as triglycerides and of the gamma-linolenic acid is > 3:1 to ≤ 200:1, preferably ≥ 20:1 to ≤ 100:1, more preferably ≥ 50:1 to ≤ 60:1, especially preferably ≥ 56:1 to ≤58:1.

9. Composition of claims 1 to 8, wherein the composition is solid, liquid and/or in the form of a gel; the composition preferably is provided in pharmaceutical forms selected from the group comprising tablets, coated tablets, dragees, capsules, powder, granulate, solutions and/or effervescent tablets.

10. Composition of claims 1 to 9, wherein the content of the pharmaceutical form has a total weight of ≥ 1 mg to ≤ 1000 mg, preferably ≥ 200 mg ≤ 800 mg, more preferably ≥ 400 mg to ≤ 700 mg, especially preferably ≥ 596 mg to ≤ 598 mg.

11. Composition of claims 1 to 10, wherein the composition contains adjuvants selected from the group comprising glycerol monostearate, lecithin and/or gelatine.

12. Composition of claims 1 to 11, wherein the pharmaceutical form is the capsule form and/or the capsule shell has a weight of ≥ 50 mg to ≤ 500 mg, preferably ≥100 mg ≤ 400 mg, more preferably ≥ 218 mg to ≤ 256 mg, especially preferably ≥ 237 mg to ≤ 238 mg.

13. Composition of claims 1 to 12, wherein the composition contains adjuvants selected from the group comprising glycerol, gelatine and/or dyes, e.g. red iron oxide.

14. Use of the composition of claims 1 to 13 for the manufacture of an agent for dietary prophylaxis and/or treatment of diseases of the eye, preferably for the treatment of the dry eye syndrome.

15. Use of the composition of claims 1 to 14 as dietary supplement and/or agent for supplementary balanced diet.

16. Agent, dietary supplement or agent for supplementary balanced diet, especially food, comprising a composition according to claims 1 to 15.

## Revendications

1. Composition pour le traitement des symptômes des yeux secs, contenant des acides gras oméga 3 et oméga 6, **caractérisée en ce que** la composition comprend des composés du zinc, de préférence sous la forme de sulfate de zinc.

2. Composition selon la revendication 1, dans laquelle la composition comporte au moins une vitamine, choisie parmi le groupe comprenant la vitamine E, la vitamine C, la vitamine B, de préférence la vitamine B6 et/ou la vitamine B12.

3. Composition selon les revendications 1 à 2, dans laquelle les acides gras oméga 3 sont présents comme triglycérides d'huile de poisson.

4. Composition selon les revendications 1 à 3, dans laquelle l'acide gras oméga 3 est un acide eicosapentaénoïque, pouvant être obtenu de préférence à partir d'huile de table, d'huile de colza, d'huile de graines de lin et/ou d'huile de poisson.

5. Composition selon les revendications 1 à 4, dans laquelle l'acide gras oméga 3 est l'acide docosahexaénoïque.

6. Composition selon les revendications 1 à 5, dans laquelle l'acide gras oméga 6 est de l'acide gamma-linolénique, pouvant être obtenu de préférence à partir de l'huile de graines de bourrache, de l'huile d'oenothère et/ou d'huile de pépins de cassis.

7. Composition selon les revendications 1 à 6, dans laquelle la composition, rapportée à une dose unique de la composition, comporte :
• ≤ 10 mg à ≤500 mg, de préférence ≥ 100 mg à ≤400 mg, encore plus préférablement ≥200 mg à ≤300 mg, de façon particulièrement préférée ≥280 mg à ≤290 mg d'acide gras oméga 3, calculés comme triglycérides ; et/ou
• ≥1 mg à ≤10 mg, de préférence ≥2 mg à ≤8 mg, encore plus préférablement ≥4 mg à ≤6 mg, de façon particulièrement préférée ≥4,5 mg à ≤5,5 mg d'acide gamma linolénique ; et/ou
• ≥1 mg à ≤5 mg, de préférence ≥2 mg à ≤4,5 mg, encore plus préférablement ≥3 mg à ≤4 mg, de façon particulièrement préférée ≥3,1 mg à ≤3,5 mg de vitamine E ; et/ou
• ≥0,01 mg à ≤5 mg, de préférence ≥0,1 mg à ≤3 mg, encore plus préférablement ≥0,5 mg à ≤2 mg, de façon particulièrement préférée ≥0,9 mg à ≤1,1 mg de mélange concentré de tocophérol ; et/ou
• ≥ 1 mg à ≤50 mg, de préférence ≥10 mg à ≤40 mg, encore plus préférablement ≥15 mg à ≤30 mg, de façon particulièrement préférée ≥19 mg à ≤2 mg de vitamine C ; et/ou
• ≥0,1 mg à ≤1,0 mg, de préférence ≥0,3 mg à ≤0,8 mg, encore plus préférablement ≥0,5 mg à ≤0,75 mg, de façon particulièrement préférée ≥0,6 mg à ≤0,7 mg de vitamine B6 ; et/ou
• ≥0,01 µg à ≤1,0 µg, de préférence ≥0,15 µg à ≤0,7 µg, encore plus préférablement ≥0,25 µg à ≤0,5 µg, de façon particulièrement préférée ≥0,32 µg à ≤0,34 µg de vitamine B12 ; et/ou
• ≥0,1 mg à ≤1,0 mg, de préférence ≥1,5 mg à ≤5 mg, encore plus préférablement ≥2 mg à ≤4 mg, de façon particulièrement préférée ≥3,3 mg à ≤3,4 mg de zinc.

8. Composition selon les revendications 1 à 7, dans laquelle le rapport pondéral des acides gras oméga 3, calculés comme triglycérides, et de l'acide gamma linolénique est de ≥3 : 1 à ≤200 : 1, de préférence ≥20 : 1 à ≤100 : 1, encore plus préférablement ≥50 : 1 à ≤60 : 1, de façon particulièrement préférée ≥56 : 1 à ≤58 : 1.

9. Composition selon les revendications 1 à 8, dans laquelle la composition est présente sous forme solide, liquide et/ou de gel ; de préférence, la composition est présente dans des formes d'administration choisies parmi le groupe comprenant les comprimés, les comprimés pelliculés, les dragées, les gélules, la poudre, les granulés, les solutions et/ou les comprimés effervescents.

10. Composition selon les revendications 1 à 9, dans laquelle le contenu de la forme d'administration présente un poids total de ≥1 mg à≤1000 mg, de préférence de ≥200 mg à ≤800 mg, encore plus préférablement de ≥400 mg à ≤700 mg, de façon particulièrement préférée de ≥596 mg à ≤598 mg.

11. Composition selon les revendications 1 à 10, dans laquelle la composition comporte des substances auxiliaires choisies parmi le groupe comprenant le monostéarate de glycérol, la lécithine, et/ou la gélatine.

12. Composition selon les revendications 1 à 11, dans laquelle leur forme d'administration est sous forme de gélule et/ou dans laquelle l'enveloppe de la gélule présente un poids de ≥50 mg à ≤500 mg, de préférence ≥100 mg à ≤400 mg, encore plus préférablement ≥218 mg à ≤256 mg, de façon particulièrement préférée ≥237 mg à ≤238 mg.

13. Composition selon les revendications 1 à 12, dans laquelle l'enveloppe de la gélule comporte des substances auxiliaires choisies parmi le groupe comprenant le glycérol, la gélatine et/ou des colorants, par exemple de l'oxyde rouge de fer.

14. Utilisation de la composition selon les revendications 1 à 13 à la préparation d'un produit pour la prévention diététique et/ou le traitement d'affections oculaires, de préférence pour le traitement des symptômes des yeux secs.

15. Utilisation de la composition selon les revendications 1 à 14 comme complément alimentaire et/ou comme moyen de régime équilibré complémentaire.

16. Moyen, complément alimentaire ou moyen de régime équilibré complémentaire, en particulier denrée alimentaire, comprenant une composition selon les revendications 1 à 15.
